# EUROPÄISCHE PATENTSCHRIFT

(11) **EP 1 914 576 B1**
(45) Veröffentlichungstag und Bekanntmachung des Hinweises auf die Patenterteilung: **16.01.2019**
(21) Anmeldenummer: 06021726.2
(22) Anmeldetag: 17.10.2006
(51) Int. Cl.: G02B 6/02, G02B 6/24, G02B 6/26, G02B 5/32, G03H 1/04, A61B 18/22, A61N 5/06, G03H 1/02

(54) **Laserapplikator mit einem einen photorefraktiven Bereich mit Volumenhologramm umfassenden Lichtleiter.**
Laser applicator with an optical lightguide, the optical lightguide comprising a photorefractive section having a volume hologram.
Applicateur laser avec un guide d'onde optique qui comprend une section photorefractive ayant un hologramme en volume.

(43) Veröffentlichungstag der Anmeldung: 23.04.2008
(73) Patentinhaber: Dornier MedTech Laser GmbH, 82234 Wessling (DE)
(72) Erfinder: Rheinwald, Markus, 86916 Kaufering (DE); Rückle, Benno, 82343 Pöcking (DE)
(74) Vertreter: Grünecker Patent- und Rechtsanwälte PartG mbB

(56) Entgegenhaltungen:
- EP-A2- 0 435 201
- EP-A2- 0 495 605
- WO-A-98/47032
- JP-A- 2002 162 524
- US-A- 5 693 043
- US-B1- 6 398 778
- US-B1- 6 423 055
- SCHULTZ S M ET AL: "VOLUME GRATING PREFERENTIAL-ORDER FOCUSING WAVEGUIDE COUPLER", OPTICS LETTERS, OPTICAL SOCIETY OF AMERICA, vol. 24, no. 23, 1 December 1999 (1999-12-01), pages 1708-1710, XP000903974, ISSN: 0146-9592

## Beschreibung

Die Erfindung betrifft Lichtleiter umfassende Laserapplikatoren.

Mit Laserapplikatoren wird beispielsweise im medizinischen Bereich die Behandlung von biologischem Gewebe mit Laserstrahlung ermöglicht. Dabei wird beispielsweise bei der laserinduzierten Thermotherapie (LITT) eine Glasfaser zur Leitung der Laserstrahlung mit Hilfe eines Katheters unmittelbar an oder in einen zu behandelnden Gewebebereich gebracht. Das durch den Applikator in das umliegende Gewebe gestrahlte Laserlicht wird absorbiert und führt zu einer lokalen Temperaturerhöhung, die koagulative und hyperthermische Effekte hervorruft. Hierdurch kommt es zu einer sofortigen oder verzögerten Gewebsnekrose.

Lichtleiterfasern bzw. Lichtwellenleiterfasern, wie sie für Laserapplikatoren verwendet werden, bestehen üblicherweise aus Mineralglas oder organischem Glas (Kunststoff). Ein Kern wird von einem Mantel und einer Beschichtung umgeben, wobei der Mantel eine niedrigere optische Brechzahl als der Kem aufweist, so dass an der Grenzschicht zwischen Kern und Mantel Totalreflexion und somit eine Führung der Strahlung im Kern erfolgt.

Für die Abgabe oder Auskopplung der Laserstrahlung aus der Faser wird der Faserkern beispielsweise an bestimmten Stellen geeignet angeraut, so dass in diesen Bereichen eine homogene Abstrahlung erzielt wird. Auf diese Weise wird die Laserstrahlung divergent vom Applikator abgestrahlt, so dass die Energiedichte an der Katheteroberfläche am größten ist und mit zunehmendem Abstand zum Applikator stark abfällt. Damit wirkt die Laserstrahlung im Nahbereich des Applikators am stärksten, was die Gefahr einer Überhitzung bzw. Verbrennung an der Applikatoroberfläche nach sich zieht.

Eine azimutale Asymmetrie wird nach dem Stand der Technik durch eine halbseitige Verspiegelung des Katheters erreicht. Alternativ dazu werden Katheter verwendet, in deren Innerem bewegliche Spiegel angeordnet sind, die während einer Bewegung des Katheters geschwenkt werden, so dass die Laserstrahlen zeitlich nacheinander auf den gleichen Ort treffen. Mit dieser Anordnung kann eine azimutale Asymmetrie durch eine Beschränkung des Bewegungsbereiches eines Schwenkspiegels erreicht werden: Allerdings geht während der Bewegung des Katheters oder des Schwenkspiegels Wärmeenergie durch Wärmeleitung verloren, so dass die Wirksamkeit bei dieser Art der Behandlung verschlechtert wird. Außerdem ist der Spiegelaufbau aufwändig und störanfällig. Dokument US 6,398,778 B1 offenbart einen Laserapplikator, umfassend einen Lichtleiter mit einem photorefraktiven Bereich, an einem Ende des Lichtleiters, wobei in dem photorefraktiven Bereich ein Bragg-Gitter derart ausgebildet ist, dass in den Lichtleiter eingekoppeltes Licht durch das Bragg-Gitter unter einem vorherbestimmten Winkel auskoppelbar ist.

Angesichts dieses Stands der Technik ist es ein Ziel der vorliegenden Erfindung, einen einfach herzustellenden und zuverlässigen Laserapplikator für die Behandlung von biologischem Gewebe mit Laserstrahlung, für Laserapplikationen beispielsweise im medizinischen Bereich, bereitzustellen, bei dem eingekoppelte Strahlung in gewünschter und effizienter Weise ausgekoppelt und geformt werden kann. Hierbei soll eine lokale Überhitzung an der Katheteroberfläche oder an der Faseroberfläche vermieden werden.

Dieses Ziel wird erreicht durch einen Laserapplikator gemäß Patentanspruch 1.

Es hat sich überraschenderweise herausgestellt, dass die Verwendung derartiger Volumenhologramme die Auskopplung von Licht in gewünschter Weise zuverlässig erlaubt. Ein solcher Lichtleiter weist keinen komplizierten Aufbau auf und ist somit nicht störanfällig. Je nach gewünschter Anwendung kann somit ein entsprechender Lichtleiter bereitgestellt werden, mit dem eine Auskopplung des Lichts auf einen bestimmten Fokus erreicht wird.

Photorefraktiv bedeutet, dass sich unter Lichteinfluss der Brechungsindex dauerhaft ändert. Ein photorefraktives Material ist somit photosensitiv. Durch die Verwendung von photorefraktiven Materialien lässt sich in einfacher Weise ein Volumenhologramm, insbesondere ein Volumenphasenhologramm, herstellen. Ein Volumenhologramm ist ein holographisches Gitter, das eine nicht zu vernachlässigende Ausdehnung in drei Dimensionen, also auch insbesondere in der Ausbreitungsrichtung der Lichtstrahlen, besitzt.

Der photorefraktive Bereich ist an einem Ende des Lichtleiters vorgesehen. Dies ermöglicht die Auskopplung von Licht, insbesondere an schwer zugänglichen Stellen.

Der photorefraktive Bereich umfasst ein anderes Material als der Rest des Lichtleiters. Dies erlaubt es, die Materialien für den Lichtleiter je nach Bereich geeignet zu wählen. So kann beispielsweise für den photorefraktiven Bereich ein Material mit für das Volumenhologramm besonders vorteilhaften optischen Eigenschaften gewählt werden und für den Rest des Lichtleiters ein Material, das zum einen die für die Lichtleitungseigenschaft erforderlichen Merkmale und ggf. eine hohe Flexibilität aufweist.

Der photorefraktive Bereich kann in einem Abschnitt des Lichtleiters ausgebildet sein, der mit dem Rest des Lichtleiters zerstörungsfrei oder nicht zerstörungsfrei lösbar verbunden ist. Der Abschnitt mit dem photorefraktiven Bereich kann insbesondere mit dem Rest des Lichtleiters verschmolzen sein.

Der photorefraktive Bereich kann im Kern des Lichtleiters ausgebildet sein. Insbesondere kann der Kern des gesamten Lichtleiters ein photorefraktives Material umfassen.

Der photorefraktive Bereich kann verschiedenste anorganische und organische Materialien umfassen. Er kann insbesondere ein Germanium-dotiertes Glas umfassen. Der photorefraktive Bereich kann zusätzlich oder alternativ eine Dotierung mit Wasserstoff aufweisen, insbesondere in Form eines Wasserstoff-dotierten Glases. Weitere geeignete Materialien werden beispielsweise in E. Mecher, "Erhöhung der Sensitivität photorefraktiver holographischer Speichermedien auf Basis von amorphen organischen Materialien", Dissertation, LMU München, 2001, genannt.

Der Lichtleiter kann als Lichtleiterfaser ausgebildet sein. Lichtleiterfasern, die damit einen photorefraktiven Bereich mit einem Volumenhologramm umfassen, sind besonders geeignet, beispielsweise in der Medizin bei minimalinvasiven Verfahren eingesetzt zu werden. Weiterhin wird mit solchen Lichtleiterfasern auch die Bearbeitung anorganischer Materialien insbesondere in Hohlräumen vereinfacht.

Bei den zuvor beschriebenen Lichtleitern kann der vorherbestimmte Fokus einen Punktfokus, einen Linienfokus, einen Ringfokus oder einen Dreiecksfokus umfassen. Weiterhin kann der vorherbestimmte Fokus die Form einer Kombination aus einem Punkt, einer Linie, einem Ring und/oder einem Dreieck aufweisen. Der Fokus lässt sich somit auf die Art der gewünschten Verwendung des Lichtleiters anpassen.

Insbesondere kann der vorherbestimmte Fokus einen Ringfokus mit der optischen Achse des Lichtleiters als Symmetrieachse umfassen. Unter des optischen Achse eines Lichtleiters wird die Achse verstanden, entlang der das Licht im Lichtleiter geleitet wird. Bei einer Lichtleiterfaser bildet die Faser selbst die Symmetrieachse.

Der Lichtleiter kann zum Leiten von Laserstrahlung, insbesondere im infraroten Bereich, ausgebildet sein. Insbesondere infrarote Laserstrahlung ist für die Laserinduzierte Thermotherapie geeignet.

Der photorefraktive Bereich kann insbesondere am distalen Ende des Laserapplikators vorgesehen sein.

Weiterhin stellt die Erfindung ein Verfahren zum Herstellen eines Laserapplikators wie zuvor beschrieben, mit den Schritten bereit:
Bereitstellen eines Lichtleiters mit einem photorefraktiven Bereich,
Ausbilden eines Volumenhologramms in dem photorefraktiven Bereich, so dass in den Lichtleiter eingekoppeltes Licht durch das Volumenhologramm mit einem vorherbestimmten Fokus auskoppelbar ist.

Das Bereitstellen kann die Schritte umfassen:
Bereitstellen eines Lichtleiterelements und eines Elements umfassend den photorefraktiven Bereich,
Verbinden des Elements mit dem Lichtleiterelement,
wobei das Volumenhologramm vor oder nach dem Verbinden ausgebildet wird. Insbesondere im Falle einer Lichtleiterfaser kann das Bereitstellen eines Elements mit photorefraktivem Bereich ein Bereitstellen eines weiteren Lichtleiterelements, insbesondere mit photorefraktivem Faserkem, umfassen.

Das Verbinden des Lichtleiterelements und des photorefraktiven Elements kann ein Verschmelzen umfassen. Andere Verbindungsarten, insbesondere auch zum zerstörungsfrei lösbaren Verbinden, sind allerdings ebenfalls möglich.

Das Ausbilden eines Volumenhologramms kann ein Bestrahlen mit zwei Teilstrahlen von UV-Laserlicht umfassen, wobei ein Teilstrahl dem auszubildenden Volumenhologramm entsprechend modifiziert wird.

Weitere Merkmale und Vorteile der Erfindung werden nachfolgend an Hand der Figuren beschrieben. Dabei illustriert
- Figur 1: schematisch ein Verfahren zu Herstellung eines Lichtleiters mit einem Volumenhologramm
- Figur 2: schematisch die Verwendung eines Beispiels eines Lichtleiters mit einem Volumenhologramm.

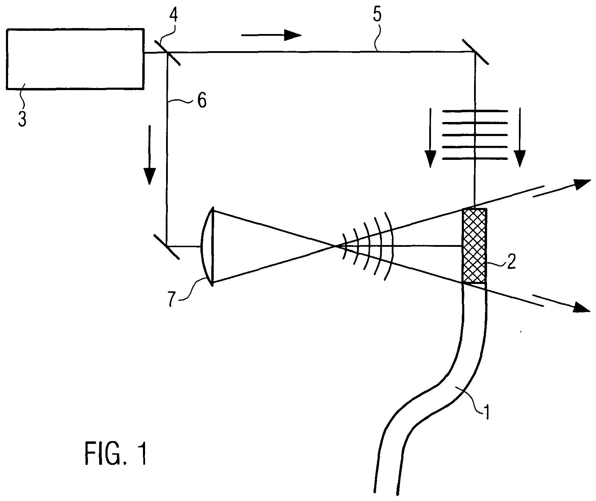

In Figur 1 wird beispielhaft die Herstellung eines Volumenhologramms am distalen Ende einer Lichtleiterfaser illustriert. Dabei wird eine Lichtleiterfaser 1 mit einem Abschnitt 2 mit einem photorefraktiven Bereich bereitgestellt.

Die Lichtleiterfaser kann beispielsweise eine Glasfaser mit einem Faserkern aus Glas sein. An ihrem distalen Ende, dem Abschnitt 2, ist sie photosensibilisiert. Dieser Abschnitt mit dem photorefraktiven Bereich kann beispielsweise ein Stück Lichtleiterfaser mit einem Faserkern aus Germanium-dotierten Glas sein. Der Faserkern kann weiterhin mit Wasserstoff dotiert sein. Dieser Abschnitt wird dann mit dem Rest der Lichtleiterfaser verbunden, beispielsweise durch Verschmelzen.

In dem photorefraktiven Bereich wird ein Volumenhologramm durch Bestrahlung mit zwei UV-Laserteilstrahlen erzeugt. Hierfür wird eine UV-Lichtquelle (3) bereitgestellt, die einen Laserstrahl im UV-Bereich ausstrahlt.

Als UV-Lichtquelle können beispielsweise gepulste Excimer-Laser mit Wellenlängen von 193 nm bzw. 248 nm, ein frequenzverdoppelter Argon-Ionenlaser (500 mW, cw) mit einer Wellenlänge von 244 nm oder ein frequenzvervierfachter Nd:YVO4-Laser (100 mW, cw) mit einer Wellenlänge von 266 nm eingesetzt werden.

Mit Hilfe eines Strahlteilers 4 wird der Laserstrahl in zwei Teilstrahlengänge (5) und (6) aufgeteilt. Der eine Teilstrahl 5 wird mit näherungsweise ebenen Wellenfronten am distalen Ende in die Lichtleitfaser eingekoppelt.

Der andere Teilstrahl 6 wird entsprechend dem auszubildenden Volumenhologramm bzw. dem gewünschten Auskopplungsverhalten durch Linsen und/oder Spiegel modifiziert. In dem gezeigten Beispiel wird der zweite Teilstrahl 6 mittels einer Linse 7 auf einen Punkt mit einem vorherbestimmten Abstand von der Lichtleitfaser fokussiert. Somit wird ein Punktfokus erzeugt. Danach fällt der Laserstrahl auf den photorefraktiven Bereich 2.

In dem photorefraktiven Bereich des Abschnitts 2 interferieren die beiden Teilstrahlen 5 und 6 und erzeugen ein Volumenphasenhologramm. Vom Laser 3 bis zum Überlagerungsbereich müssen beide Teilstrahlengänge die gleiche optische Weglänge haben. Wenn der gesamte Faserkern photorefraktiv ist, kann dieses Einschreiben des Volumenhologramms in den Abschnitt 2 grundsätzlich sowohl nach als auch während des Ziehprozesses der Lichtleitfaser erfolgen. Bei der Verwendung eines Lichtleiterelements ohne photorefraktiven Faserkern und eines zusätzlichen Faserelements mit photorefraktivem Kern, die beide miteinander verbunden werden, kann entsprechend das Einschreiben grundsätzlich vor oder nach dem Verbinden durchgeführt werden. Vorzugsweise erfolgt es jedoch nach dem Verbinden, da dann die relative Anordnung der beiden Lichtleiterelemente fixiert ist.

Bei der Erzeugung eines Volumenhologramms, das bei einer anschließenden Verwendung der Lichtleiterfaser das Licht unter einem anderen Winkel oder auf einen anderen Fokus als in der Figur gezeigt auskoppeln soll, ist der zweite Teilstrahl 6 entsprechend zu modifizieren. Statt eines Punktfokus kann somit beispielsweise auch ein Linienfokus, ein Ringfokus oder ein Dreiecksfokus erzeugt werden. Weiterhin ist auch eine Überlagerung mehrerer dieser Fokusse möglich.

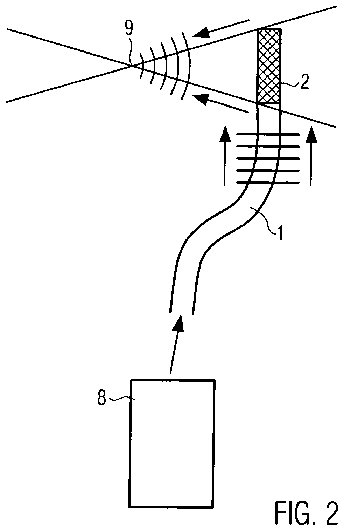

Figur 2 illustriert schematisch den Betrieb eines Lichtleiters, an dessen distalem Ende ein Volumenhologramm ausgebildet ist. Gezeigt ist ein aus dem Verfahren gemäß Figur 1 entstandenen Lichtleiter 1 mit einem Abschnitt 2 mit photorefraktivem Bereich, in dem ein Volumenhologramm für einen Punktfokus ausgebildet ist.

Im Anwendungsbetrieb wird der Strahl eines Lasers 8 proximal in die Faser 1 eingekoppelt und erreicht somit den Bereich des Volumenhologramms aus der entgegengesetzten Richtung im Vergleich zum Einschreiben des holographischen Gitters gemäß Figur 1.

Je nach gewünschter Anwendung kann als Laser 8 beispielsweise ein Nd:YAG-Laser mit einer Wellenlänge von 1064 nm oder ein Diodenlaser mit einer Wellenlänge zwischen 800 und 1000 nm eingesetzt werden. Geeignete Laserleistungen können beispielsweise zwischen 5 und 100 W liegen.

Wenn der eingekoppelte Strahl den am distalen Ende befindlichen Streubereich erreicht, wird er durch das Volumenhologramm derart gebeugt, dass der Strahlenverlauf des zweiten Teilstrahls 6 von Figur 1 in umgekehrter Richtung erhalten wird. Somit wird das eingekoppelte Licht im Abschnitt des photorefraktiven Bereichs aus der Lichtleitfaser ausgekoppelt und weist einen Punktfokus 9 auf. Die Effizienz der Strahlumformung in dem Volumenhologramm hängt dabei insbesondere von den Kohärenzeigenschaften des verwendeten Lasers 8 ab.

Derartige Lichtleiter, insbesondere Lichtleiterfasern, können in verschiedensten Bereichen angewendet werden. Bei medizinischen Anwendungen kann beispielsweise eine Lichtleitfaser in eine natürliche Körperöffnung eingeführt oder mit Hilfe eines Endoskops oder Katheters an eine Behandlungsstelle gebracht werden. Durch die unter einem beliebigen Winkel und mit einem beliebigen Fokus ausgekoppelte Laserstrahlung kann Gewebe in schwer zugänglichen Bereichen abgetragen oder koaguliert werden. Somit eignet sich ein derartiger Lichtleiter insbesondere bei interstitiellen Behandlungen, für die Gastroenterologie, Urologie oder Angioplastie.

## Patentansprüche

1. Laserapplikator für die Behandlung von biologischem Gewebe mit Laserstrahlung, umfassend einen Lichtleiter (1) mit wenigstens einem photorefraktiven Bereich, wobei in dem photorefraktiven Bereich ein Volumenhologramm derart ausgebildet ist, dass in den Lichtleiter eingekoppeltes Licht durch das Volumenhologramm mit einem vorherbestimmten Fokus auskoppelbar ist,
wobei der photorefraktive Bereich an einem Ende des Lichtleiters vorgesehen ist,
wobei der photorefraktive Bereich ein anderes Material als der Rest des Lichtleiters umfasst.

2. Laserapplikator nach Anspruch 1, wobei der photorefraktive Bereich ein Germanium-dotiertes Glas umfasst.

3. Laserapplikator nach einem der vorangegangenen Ansprüche, wobei der photorefraktive Bereich ein Wasserstoff-dotiertes Glas umfasst.

4. Laserapplikator nach einem der vorangegangenen Ansprüche, wobei der Lichtleiter als Lichtleiterfaser ausgebildet ist.

5. Laserapplikator nach einem der vorangegangenen Ansprüche, wobei der vorherbestimmte Fokus einen Punktfokus umfasst.

6. Laserapplikator nach einem der vorangegangenen Ansprüche, wobei der vorherbestimmte Fokus einen Linienfokus umfasst.

7. Laserapplikator nach einem der vorangegangenen Ansprüche, wobei der vorherbestimmte Fokus einen Dreiecksfokus umfasst.

8. Laserapplikator nach einem der vorangegangenen Ansprüche, wobei der vorherbestimmte Fokus die Form einer Kombination aus einem Punkt, einer Linie, einem Ring und/oder einem Dreieck aufweist.

9. Laserapplikator nach einem der vorangegangenen Ansprüche, wobei der vorherbestimmte Fokus einen Ringfokus mit der optischen Achse des Lichtleiters als Symmetrieachse umfasst.

10. Laserapplikator nach einem der vorangegangenen Ansprüche, wobei der Lichtleiter zum Leiten von Laserstrahlung, insbesondere im infraroten Bereich, ausgebildet ist.

11. Verfahren zum Herstellen eines Laserapplikators nach einem der Ansprüche 1 - 10, mit den Schritten:
Bereitstellen eines Lichtleiters mit einem photorefraktiven Bereich,
Ausbilden eines Volumenhologramms in dem photorefraktiven Bereich, so dass in den Lichtleiter eingekoppeltes Licht durch das Volumenhologramm mit einem vorherbestimmten Fokus auskoppelbar ist.

12. Verfahren nach Anspruch 11, wobei das Bereitstellen eines Lichtleiters die Schritte umfasst:
Bereitstellen eines Lichtleiterelements und eines Elements umfassend den photorefraktiven Bereich,
Verbinden des Elements mit dem Lichtleiterelement,
wobei das Volumenhologramm vor oder nach dem Verbinden ausgebildet wird.

13. Verfahren nach Anspruch 12, wobei das Verbinden ein Verschmelzen umfasst.

14. Verfahren nach einem der Ansprüche 11 - 13, wobei das Ausbilden eines Volumenhologramms ein Bestrahlen mit zwei Teilstrahlen von UV-Laserlicht umfasst, wobei ein Teilstrahl dem auszubildenden Volumenhologramm entsprechend modifiziert wird.

## Claims

1. Laser applicator for the treatment of biological tissue with laser radiation, comprising a light guide (1) with at least one photorefractive area, wherein a volume hologram is formed in the photorefractive area in such a way that light coupled into the light guide can be decoupled by the volume hologram with a predetermined focus,
wherein the photorefractive area is provided at one end of the light guide,
wherein the photorefractive area comprises a material other than that of the rest of the light guide.

2. Laser applicator according to claim 1, wherein the photorefractive area comprises a germanium-doped glass.

3. Laser applicator according to one of the preceding claims, wherein the photorefractive area comprises a hydrogen-doped glass.

4. Laser applicator according to one of the preceding claims, wherein the light guide is formed as an optical fibre.

5. Laser applicator according to one of the preceding claims, wherein the predetermined focus comprises a point focus.

6. Laser applicator according to one of the preceding claims, wherein the predetermined focus comprises a line focus.

7. Laser applicator according to one of the preceding claims, wherein the predetermined focus comprises a triangle focus.

8. Laser applicator according to one of the preceding claims, wherein the predetermined focus has the form of a combination of a point, line, ring and / or triangle.

9. Laser applicator according to one of the preceding claims, wherein the predetermined focus comprises a ring focus with the optical axis of the light guide as the axis of symmetry.

10. Laser applicator according to one of the preceding claims, wherein the light guide is formed for guiding laser radiation, particularly in the infrared range.

11. Method for producing a laser applicator according to one of claims 1 to 10, with the steps:
providing a light guide with a photorefractive area, and
developing a volume hologram in the photorefractive area, so that light coupled into the light guide can be decoupled by the volume hologram with a predetermined focus.

12. Method according to claim 11, wherein providing a light guide comprises the steps:
providing a light guide element and an element comprising the photorefractive area, and connecting the element to the light guide element,
wherein the volume hologram is formed before or after the connecting.

13. Method according to claim 12, wherein the connecting comprises a fusing.

14. Method according to one of claims 11 to 13, wherein the formation of a volume hologram comprises an irradiation with two partial beams of UV laser light, wherein a partial beam is modified according to the volume hologram to be formed.

## Revendications

1. Applicateur laser pour le traitement de tissus biologiques par un rayonnement laser, comprenant un guide d'onde optique (1) avec au moins une zone photoréfractive, un hologramme volumique étant formé dans la zone photoréfractive de manière telle que de la lumière envoyée dans le guide d'onde optique puisse être découplée par l'hologramme volumique, avec un foyer prédéterminé, applicateur laser
dans lequel la zone photoréfractive est prévue à une extrémité du guide d'onde optique, et
dans lequel la zone photoréfractive comprend un autre matériau que le restant du guide d'onde optique.

2. Applicateur laser selon la revendication 1, dans lequel la zone photoréfractive comprend un verre dopé au germanium.

3. Applicateur laser selon l'une des revendications précédentes, dans lequel la zone photoréfractive comprend un verre dopé à l'hydrogène.

4. Applicateur laser selon l'une des revendications précédentes, dans lequel le guide d'onde optique est réalisé en tant que fibre optique.

5. Applicateur laser selon l'une des revendications précédentes, dans lequel le foyer prédéterminé comprend un foyer sous forme de point.

6. Applicateur laser selon l'une des revendications précédentes, dans lequel le foyer prédéterminé comprend un foyer sous forme de ligne.

7. Applicateur laser selon l'une des revendications précédentes, dans lequel le foyer prédéterminé comprend un foyer sous forme de triangle.

8. Applicateur laser selon l'une des revendications précédentes, dans lequel le foyer prédéterminé présente la forme d'une combinaison constituée d'un point, d'une ligne, d'un anneau et/ou d'un triangle.

9. Applicateur laser selon l'une des revendications précédentes, dans lequel le foyer prédéterminé comprend un foyer sous forme d'anneau avec l'axe optique du guide d'onde optique comme axe de symétrie.

10. Applicateur laser selon l'une des revendications précédentes, dans lequel le guide d'onde optique est réalisé pour guider un rayonnement laser, notamment dans le domaine infrarouge.

11. Procédé pour la fabrication d'un applicateur laser selon l'une des revendications 1 - 10, comprenant les étapes suivantes :
fourniture et préparation d'un guide d'onde optique avec une zone photoréfractive,
formation d'un hologramme volumique dans la zone photoréfractive, de manière telle que de la lumière envoyée dans le guide d'onde optique puisse être découplée par l'hologramme volumique, avec un foyer prédéterminé.

12. Procédé selon la revendication 11, d'après lequel la fourniture et la préparation du guide d'onde optique comprend les étapes consistant à fournir et préparer un élément de guide d'onde optique et un élément comprenant la zone photoréfractive, assembler l'élément à l'élément de guide d'onde optique, l'hologramme volumique étant formé avant ou après l'opération de liaison.

13. Procédé selon la revendication 12, d'après lequel l'assemblage comprend une fusion.

14. Procédé selon l'une des revendications 11 - 13, d'après lequel la formation d'un hologramme volumique comprend une irradiation avec deux rayonnements partiels de lumière laser-UV, un rayonnement partiel étant modifié conformément à l'hologramme volumique à former.
